(19) Europäisches Patentamt
European Patent Office
Office européen des brevets

(11) Publication number: 0 325 463 A2

# (12) EUROPEAN PATENT APPLICATION

(21) Application number: 89300518.1

(22) Date of filing: 19.01.89

(51) Int. Cl.4: C 07 C 69/675
C 11 C 3/00, A 23 L 1/29,
A 23 D 5/00

(30) Priority: 19.01.88 US 144962

(43) Date of publication of application:
26.07.89 Bulletin 89/30

(84) Designated Contracting States:
AT BE CH DE ES FR GB GR IT LI LU NL SE

(71) Applicant: NABISCO BRANDS, INC.
100 DeForest Avenue
East Hanover New Jersey 07936 (US)

(72) Inventor: Klemann, Lawrence P
196 Tanglewood Drive
Sommerville, New Jersey 08876 (US)

Finley, John W
3 Old Stone Lane
Whippany, New Jersey 07981 (US)

(74) Representative: Thomas, Roger Tamlyn et al
D. Young & Co. 10 Staple Inn
London WC1V 7RD (GB)

## (54) Low calorie fat mimetics.

(57) Novel fat mimetic compositions are disclosed as reduced calorie food components. These compositions comprise complex linked esters including two polyfunctional ethers or esters linked by a polycarboxy function having a common aliphatic group. These complex linked esters may be partially broken down in the body to produce digestion residues which are substantially non-digestible themselves and are sufficiently hydrophilic to enable the digestion residues to be incorporated in the normal stool. These fat mimetic compounds are useful as replacements for fats and oils for most food applications.

EP 0 325 463 A2

Description

# LOW CALORIE FAT MIMETICS

## BACKGROUND OF THE INVENTION

The present invention relates to edible fat mimetic materials, and especially to new compounds which have a desireable combination of properties and their use in edible compositions.

Obesity is perceived as a common problem in contemporary society. This condition is due, in many people, to a greater intake of calories than are expended. While genetic and behavioral factors play a major role, it is generally agreed that reasonable modifications of the caloric value of foods can be valuable in reaching a more desirable equilibrium weight for an individual predisposed to obesity.

Many foods which provide gustatory satisfaction contain significant fat levels. This can be a problem for individuals drawn to these foods because fat has about twice the caloric density of protein and carbohydrates. It has, in fact, been estimated that fat contributes about 40% of the total calories in the diet. It has long been desired to reduce the available calories of dietary fat without decreasing the appeal or satiety expected of fatty foods. It has been reported that this would offer a convenient and practical method by which obesity could be controlled, ideally without requiring a dieter to restrict total food intake.

Unfortunately, of the materials heretofore suggested as fat replacements, few have all of the desirable attributes of natural triglyceride fats and oils. One approach to lower the caloric value of edible fat has been to decrease the amount of triglyceride that is absorbed in the human system since the usual edible triglyceride fats are almost completely absorbed (see *Lipids,* Vol. II, H.J. Deuel, Interscience Publishers, Inc., New York 1955, page 215). The absorbability of triglyceride fat could be decreased by altering either the alcohol or the fatty acid portion of the molecule. There have been some experiments that have demonstrated a decrease in absorbability with certain fatty acids; for example, erucic acid (H. J. Deuel, A.L.S. Cheng, and M. G. Morehouse, *Journal of Nutrition,* Vol. 35 [1948], page 295, and stearic acid if present as tristearin (F. H. Mattson, *Journal of Nutrition,* Vol. 69 [1959], page 338. Also, U.S. Patent 2,962,419, to Minich discloses that fatty acid esters which contain a neopentyl nucleus are not digested like normal fats and thus can be used as fat substitutes in food compositions.

Several other patents disclose edible compounds which are not digested or absorbed to the same extent as natural triglycerides. In U.S. Patent 3,579,548, White discloses certain glycerol esters of certain branched carboxylic acids which are said to have these properties. And, in U.S. Patent 3,600,186, Mattson and Volpenhein disclose sugar and sugar alcohol fatty acid esters having at least four fatty acid ester groups. All of these compounds are said to possess physical properties similar to ordinary triglyceride fat, but to be absorbed less readily when eaten. It is, unfortunately, this very attribute which causes undesirable and potentially embarrassing side effects, including the frank anal discharge of the materials.

In a greater departure from conventional glyceride ester chemistry, Canadian Patent 1,106,681 to Trost discloses glycerol dialkyl ether compounds which are said to have functional properties similar to those of conventional fats, but which are not absorbed in the digestive tract to any significant degree. Also, Ward, Gros and Feuge have reported in New Fat Products: Glyceride Esters of Adipic Acid; *JAOCS,* Vol. 36 [1959], page 667 that highly viscous oils formed by reacting two glycerol molecules with a diabasic acid, such as fumaric, succinic and adipic acids, and then reacting one of the hydroxyl groups of each glycerol moiety with a fatty acid, are useful in the food industry, primarily as lubricants and coatings.

In U.S. Patent 4,508,746, Hamm discloses a low-calorie substitute for at least a portion of the edible oil component in oil-based food compositions which low-calorie substitute is comprised in substantial proportion of at least one low-calorie oil component selected from the group consisting of thermally stable polycarboxylic acids having 2 to 4 carboxylic acid groups esterified with saturated or unsaturated alcohols having straight or branched carbon chains of from 8 to 30 carbon atoms. See also D. J. Hamm; Preparation and Evaluation of Trialkoxytricarballylate, Trialkoxycitrate, Trialkoxyglycerylether, Jojoba Oil, and Sucrose Polyester as Low Calories Replacements of Edible Fats and Oils; *J. of Food Science,* Vol. 49 [1984], pages 419-426.

In another attempt at stimulating the natural properties of fat, Fulcher discloses certain diesters in U.S. Patent 4,582,927. These compounds have at least two carboxylate groups joined to a common carbon atom, with each of the carboxylate groups containing the residue of a 12 to 18 carbon alkyl, alkenyl or dienyl alcohol.

One of the main problems in attempting to formulate fat-like compounds that have decreased absorbability and thus low caloric properties is to maintain the desirable and conventional physical properties of edible fat. Thus, to be a practical low calorie fat, a compound must mimic conventional triglyceride fat by affording the same utility in various fat-containing food compositions such as shortening, margarine, cake mixes, and the like, and be useful in frying or baking. Unfortunately, none of the prior attempts has been successful to the degree that commercial products employing them have either been approved for safety or achieved general public acceptance in their featured role.

Among the problems with some non-absorbable fat-like materials is the possibility that they will leach fat-soluble vitamins and minerals from the body and that they function, when used in larger amounts, as purgatives. Many attempts have been made to solve these and related problems; however, a better solution would employ chemistry more compatible with the human digestive process, while providing a significant

decrease in caloric density *vis-a-vis* glyceride fats.

## SUMMARY OF THE INVENTION

The present invention provides a new class of fat mimetic compounds, new food compositions which contain them, and the process of employing these compounds in food compositions. These compositions comprise complex linked esters including two esters or ethers linked by a carboxylate ester function having a common aliphatic group. These complex linked esters are partially broken down in the body to produce digestion residues which are substantially non-digestible themselves and are sufficiently hydrophilic to enable the digestion residues to be incorporated in the normal stool. These far mimetic compounds are useful as replacements for fats and oils for most food applications.

The fat mimetic compounds can be defined by the following formula:

$R - ( \overset{O}{\overset{\|}{C}} - O - R')_n$

wherein R is a linking covalent bond or saturated or unsaturated aliphatic group; n is 2 to 6; and the R′ groups comprise residues defined by the following formula:

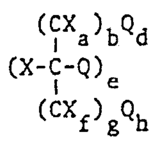

where:
C = a carbon atom;
X = a bridging bonding valence, hydrogen, or substituted or unsubstituted lower aliphatic group (e.g., $C_1$-$C_4$), the various X groups being the same of different;
Q = $-\overset{O}{\overset{\|}{C}}-O-R''$ (carboxylate),
$-R'''-\overset{O}{\overset{\|}{C}}-O-R''$ (alkylcarboxylate),
$-O-\overset{O}{\overset{\|}{C}}-R''$ (carboxy),
$-R'''-O-\overset{O}{\overset{\|}{C}}-R''$ (alkylcarboxy);
-O-R″ (alkoxy), or -R‴-O-R″ (alkylalkoxy) radicals;
with the proviso that at least one of the Q radicals be other than carboxy;
R″ = Substituted or unsubstituted aliphatic group, containing, for example, no more than 30 carbons, e.g.

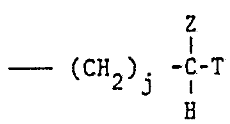

the various R′ and R″ groups, respectively, being the same or different;
R‴ = Lower alkylene, desireably methylene or ethylene, preferably methylene group, which can be the same or different;
T = Hydrogen or a substituted or unsubstituted aliphatic group, e.g., no greater than 22 carbons, containing 0 to 5 unsaturated linkages (e.g., C=C double bonds, C≡C triple bonds) per T residue;
Z = a bridging bond valence, hydrogen, or an alcohol, glycol, ester, e.g.,

$-O-\overset{O}{\overset{\|}{C}}-CH_2-T$

ether, or the like, residue;
with the proviso that there is only one bridging bonding valence per R′ group;
and where:
a = 0 to 3, preferably 0 to 2;
b = 0 to 4, preferably 0 to 1;
d = 1 or 2;
e = 0 to 5, preferably 1 to 2;

f = 0 to 3, preferably 0 to 2;
g = 0 to 4, preferably 0 to 1;
h = 1 or 2;
j = 0 to 10, preferably 0 to 3.

Preferably, each R′ group will contain from 1 to 3, most desirably 2, Q radicals.

The compounds are employed in any edible material or any food preparation process where a fat or oil (i.e., triglyceride fat) is normally employed, in total or partial replacement.

By judicious selection of the structural type, molecular size and the number of acid residues, it is possible to achieve a target reduction in calories while preferably achieving the maximum advantage from the combination of the properties of these mimetics.

## DETAILED DESCRIPTION

The following description relates to a new class of fat mimetic compounds and their incorporation into any food composition or use in conjunction with any edible material. The term "edible material" is broad and includes anything edible, whether or not intended for nutrition, e.g., it can be an additive such as an antioxidant for fats or oils, an antispatter agent, an emulsifier, or other minor functional ingredient. Thus, chewing gum, flavored coatings, oils and fats intended only for frying, and the like are included. In these, all or a portion of the usual fat is replaced by a compound of the invention.

Representative of edible materials which can contain the fat mimetic compounds of the invention in full or partial replacement of natural fat are: frozen deserts, e.g., sherbet, ice cream, ices, or milk shakes; puddings and pie fillings; margarine substitutes or blends; flavored bread or biscuit spreads; mayonnaise; salad dressing, both emulsified and non-emulsified; filled dairy products such as filled cream or filled milk; dairy or non-dairy cheese spreads; coffee lighteners, liquid and dried; flavored dips; frying fats and oils; reformed and comminuted meats; meat substitutes or extenders; whipped toppings; compound coatings; frostings and fillings; cocoa butter replacements or blends; candy, especially fatty candies such as containing peanut butter or chocolate; chewing gum; bakery products, e.g., cakes, breads, rolls, pastries, cookies, biscuits, savory crackers; mixes or ingredient premixes for any of these; as well as flavor, nutrient, drug or functional additive delivery systems.

The fat mimetics of the invention can be employed in margarine substitutes which can be either soft or hard. Margarines are generally sold as one of two principal types: namely, (1) print, hard or stick margarine and (2) soft or tub margarine. All of these products contain liquid and hard stock components which can be replaced by fat mimetics of the invention. It is an advantage of the present invention that, by eliminating some or all of the hard stock of conventional margarines, higher ratios of polyunsaturated to saturated fatty acids and lesser amounts of trans isomers can be achieved in high quality margarine products.

The fat mimetic compounds of the invention will be referred to as "complex linked esters" and can be defined by the following general formula:

$$R-(\overset{O}{\overset{\|}{C}}-O-R')_n$$

wherein the R is a linking covalent bond or saturated or unsaturated aliphatic group; preferably with up to 10 carbons most preferably 1 to 6; n is 2 to 6, and preferably 2 to 4; and the R′ groups can be the same or different and can comprise residues defined by the following formula:

$$\begin{array}{l} \quad (CX_a)_bQ_d \\ \qquad | \\ (X-C-Q)_e \\ \qquad | \\ \quad (CX_f)_gQ_h \end{array}$$

where:

C = a carbon atom;

X = a bridging bonding valence, hydrogen, or substituted or unsubstituted lower aliphatic group (e.g., $C_1$-$C_4$), the various X groups being the same of different;

Q = $-\overset{O}{\overset{\|}{C}}-O-R''$ (carboxylate),

$-R'''-\overset{O}{\overset{\|}{C}}-O-R''$ (alkylcarboxylate),

$-O-\overset{O}{\overset{\|}{C}}-R''$ (carboxy),

$-R'''-O-\overset{O}{\overset{\|}{C}}-R''$ (alkylcarboxy);

-O-R″ (alkoxy), or -R‴-O-R″ (alkylalkoxy) radicals,

with the proviso that at least one of the Q radicals be other than carboxy;
R″ = Substituted or unsubstituted aliphatic group, containing, for example, no more than 30 carbons, e.g.

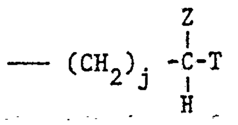

the various R′ and R″ groups, respectively, being the same or different;
R‴ = Lower alkylene, desireably methylene or ethylene, preferably methylene group, which can be the same or different;
T = Hydrogen or a substituted or unsubstituted aliphatic group, e.g., no greater than 22 carbons, containing 0 to 5 unsaturated linkages (e.g., C=C double bonds, C≡C triple bonds) per T residue;
Z = a bridging bonding valence, hydrogen, or an alcohol, glycol, ester, e.g.,

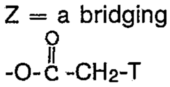

ether, or the like, residue;
with the proviso that there is only one bridging bonding valence per R′ group;
and where:
a = 0 to 3, preferably 0 to 2;
b = 0 to 4, preferably 0 to 1;
d = 1 or 2;
e = 0 to 5, preferably 1 to 2;
f = 0 to 3, preferably 0 to 2;
g = 0 to 4, preferably 0 to 1;
h = 1 or 2;
j = 0 to 10, preferably 0 to 3.

Preferably, each R′ group will contain from 1 to 3, most desirably 2, Q radicals.

Where Q groups are carboxy or alkylcarboxy, they can be fatty acid residues which are preferably derived from natural fat. The more readily digestible carboxylic acid residues can be a highly desirable essential acid or a nutritionally advantageous carboxylic acid such as: oleic, linoleic, linolenic, eicosapentaenoic acids, as well as low molecular weight carboxylic acids (e.g., acetic, propionic, butyric acids). Also suitable are other fatty acids having from about 8 to 22 carbons atoms. Examples of such fatty acids are caprylic, capric, lauric, myristic, myristoleic, palmitic, palmitoleic, stearic, ricinoleic, eleostearic, arachidic, behenic, and erucic. The fatty acids can be derived from suitable naturally-occurring or synthetic fatty acids and can be saturated or unsaturated, including positional and geometric isomers, depending on the desired physical properties, e.g., liquid or solid, of the fat compound.

Fatty acids *per se* or naturally-occurring fats and oils can serve as the source for the fatty acid component in the complex linked ester. For example, rapeseed oil provides a good source for $C_{22}$ fatty acids. $C_{16}$-$C_{18}$ fatty acids can be provided by tallow, soybean oil, or cottonseed oil. Shorter chain fatty acids can be provided by coconut, palm kernel, or babassu oils. Corn oil, lard, olive oil, palm oil, peanut oil, safflower seed oil, sesame seed oil, and sunflower seed oil, are examples of other natural oils which can serve as the source of the fatty acid component. Among the fatty acids, those that are more usually preferred have from about 14 to about 18 carbon atoms, and are most preferably selected from the group consisting of myristic, palmitic, stearic, oleic, and linoleic. Thus, natural fats and oils which have a high content of these fatty acids represent preferred sources for the fatty acid components, e.g., soybean oil, olive oil, cottonseed oil, corn oil, tallow and lard.

Where the Q groups are carboxylate, alkylcarboxylate, alkoxy or alkylalkoxy, they can be residues of fatty alcohols having saturated or unsaturated aliphatic groups (i.e., R″) with at least 5 carbons. Preferred fatty alcohols are oleyl, linoleyl, linolenyl, stearyl, palmityl, myristyl, lauryl, capryl, eicosapentaenyl, and the like. The preferred fatty alcohols are of similar chain lengths and configurations to those of the preferred fatty acids.

The particular types of fatty acids and alcohols can be selected to achieve the desired texture (both solid and liquid) and melt characteristics in the compound. Blends of complex linked esters with each other and/or with natural fats and oils and/or other fat mimetic materials such as sucrose polyesters can be selected for a desired rheology, melt profile, and mouthfeel. This is especially desirable in the case of margarine substitutes, cookie fillings, whipped toppings, etc.

Among the carboxy/carboxylate/alkoxy esters preferred for many applications are those with melting points below about 98°F because these materials melt completely in the mouth providing the organoleptic sensation of natural fats and oils. For some products, relatively sharp melting points, say in the range of from about 90° to 98°F, are desired because they provide a cooling sensation and a meltdown equivalent to high quality, solid, natural fats.

The complex linked esters may be partially broken down in the body to yield digestion residues which, preferably, are each more hydrophilic than the complex ester substrate. The majority by weight of the digestive

residues will be non-hydrolyzable by normal digestive processes, while a minor amount by weight may be susceptible to facile cleavage by the action of digestive enzymes. The selection of the exact type of chemical bonds which will provide the desired proportions of hydrolytically reactive versus "inert" sites is determined by experiment.

The following is a list of representative, but non-limiting, examples of R′ groups which can be linked to form the complex linked esters of the invention:

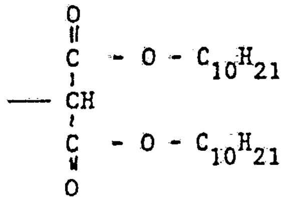

(1)

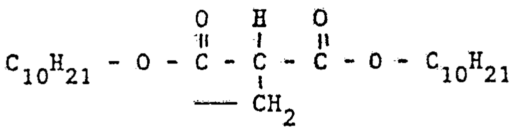

(2)

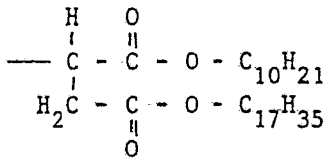

(3)

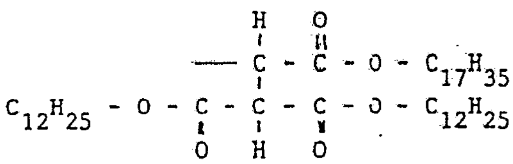

(4)

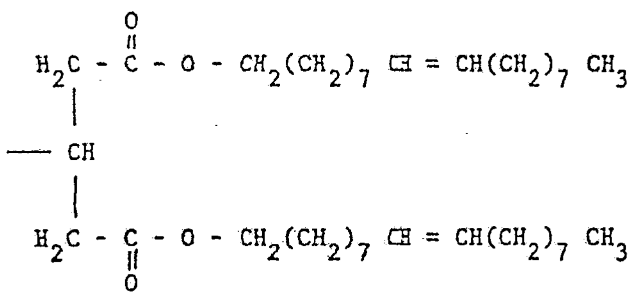

(5)

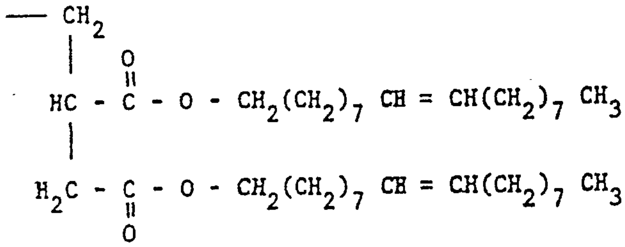

(6)

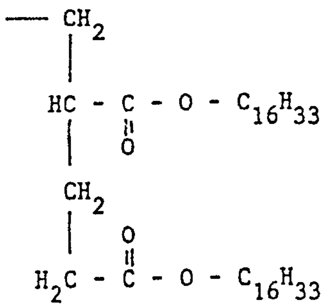

(7)

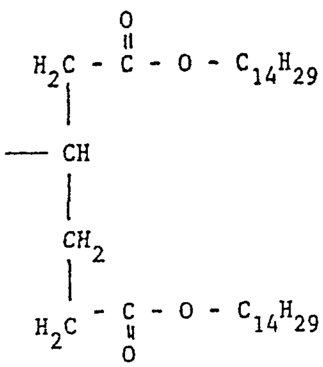

(8)

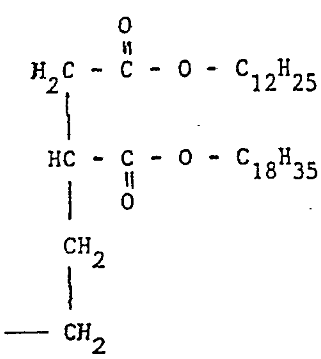

(9)

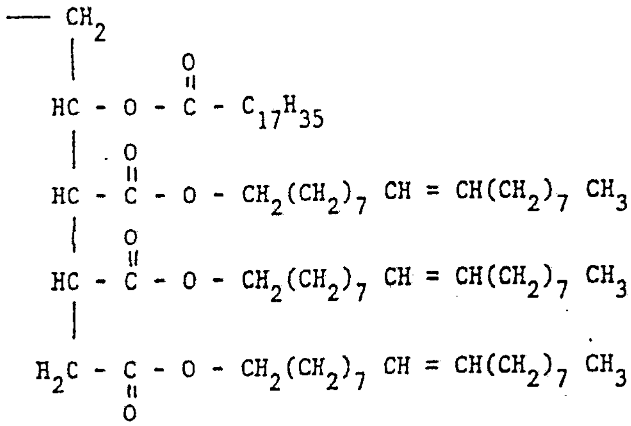

(10)

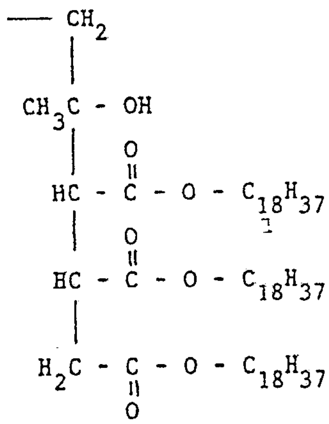

(11)

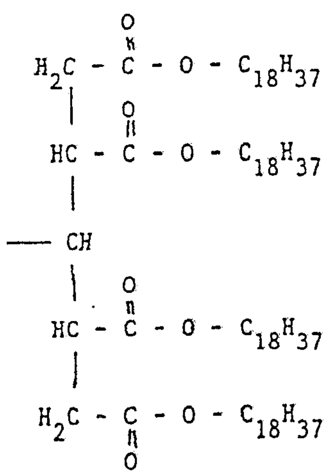

(12)

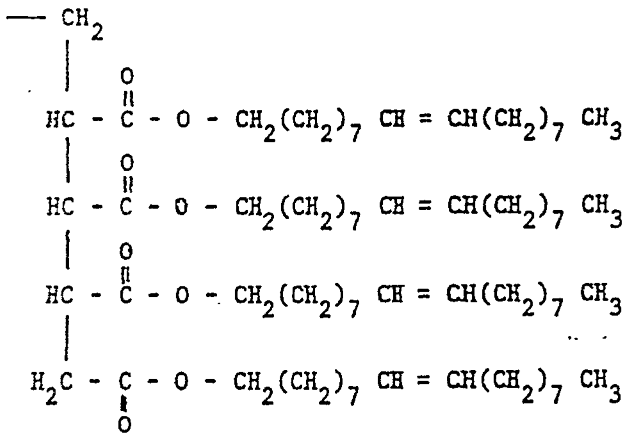

(13)

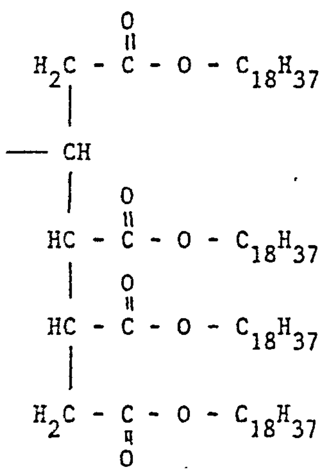

(14)

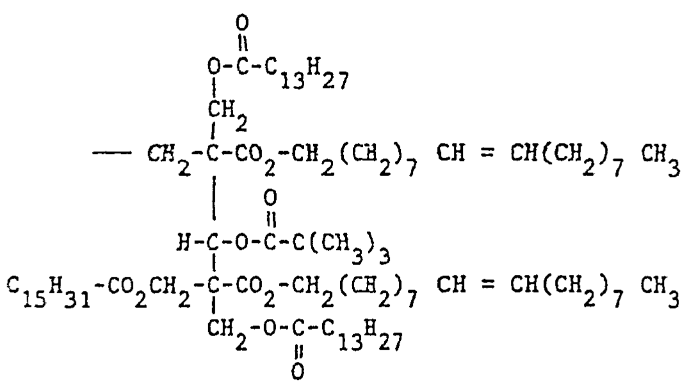

(15)

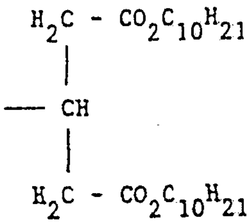

(16)

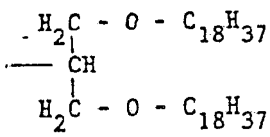

(17)

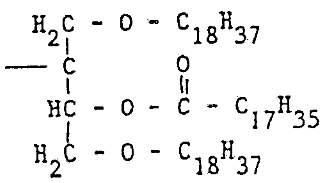

(18)

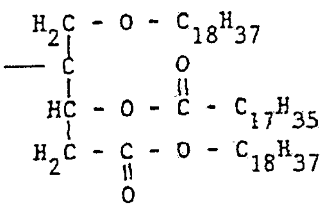

(19)

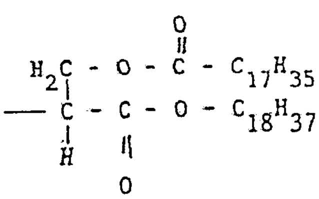

(20)

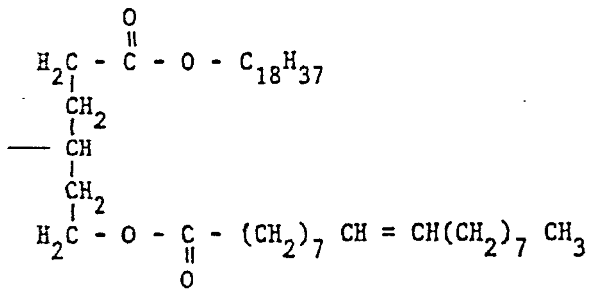

(21)

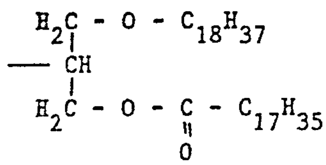

(22)

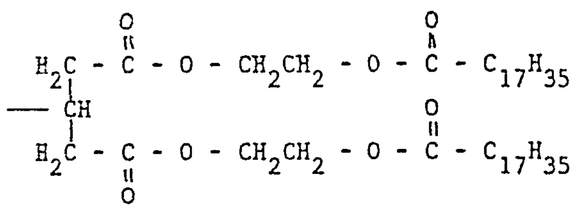

(23)

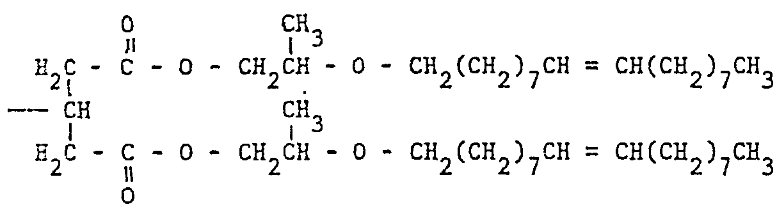

(24)

The preparation of the complex linked esters of the invention involves the reaction of alcohols of the formula R′ - OH with a polybasic compound effective to link the R′ radicals to a common molecular framework. Among the polybasic compounds are: polybasic acids of the formula

R - ( $\overset{O}{\overset{\|}{C}}$ -OH)$_n$, acid chloride of the formula

R - ( $\overset{O}{\overset{\|}{C}}$ - Cl)$_n$; alkyl esters of the formula

R - ( $\overset{O}{\overset{\|}{C}}$ - OR‴)$_n$; dibasic acid cyclic anhydrides of the formula

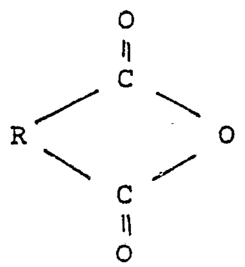 ;

or mixed anhydrides of a dibasic acid such as shown by the formula

$$R''' - \overset{O}{\overset{\|}{C}} - O - \overset{O}{\overset{\|}{C}} - R - \overset{O}{\overset{\|}{C}} - O - \overset{O}{\overset{\|}{C}} - R''';$$

wherein R, R′, R‴ and n are as defined above. The reactions will typically proceed at approximately ambient or reduced pressure and at temperatures of from about 0° to 170° C. Solvents and/or catalysts may be employed to adjust the reaction rate and product recovery as desired.

Representative dibasic acids are saturated acids such as oxalic, malonic, succinic, glutaric, adipic and unsaturated acids such as maleic, fumaric, citraconic, mesaconic, glutamic, aspartic, sebacic, and suberic. Representative of tribasic acids are citric, tricarballylic, cis, cis-1,3,5-cyclohexanetricarboxylic, cis-aconitic and trans- aconitic acids. Representative tetrabasic acids are methane tetracarboxylic acid and 1,1,2,2,-ethane tetracarboxylic acid. Suitable anhydrous acid chloride, acid bromide, and lower alkyl ester forms of these acids can be employed as desired.

The following examples are presented to further illustrate and explain the present invention and should not be taken as limiting in any regard. Unless otherwise indicated, all parts and percentages are by weight.

## EXAMPLE 1

This example shows the preparation, from intermediates to final molecular composition, of a fat mimetic material of the invention, having the following structure:

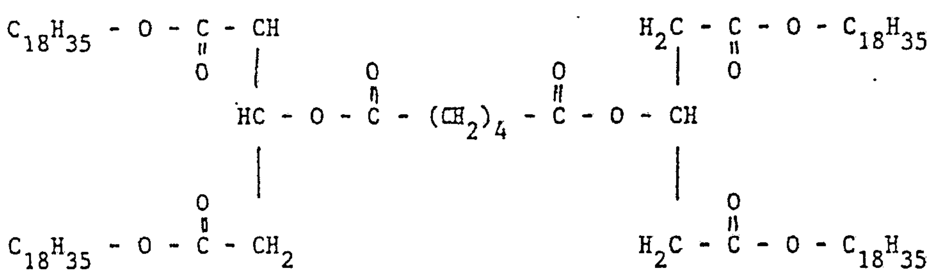

Dioleyl 3-oxyglutarate. Trichloroacetic acid (9.8g, 0.06 mole), dimethyl 1,3-acetonedicarboxylate (139.3 g, 0.80 mole), and oleyl alcohol (451.1 g, 1.68 mole, 5 mole % excess) are combined in a 2000 mL flask fitted with a distillation head, thermometer, and Teflon coated stirrer bar. The apparatus is evacuated to about 150 mmHg and is heated at 130 to 140 degrees C for 17 hours. A quantitative yield of clear orange oil is obtained. An nmr spectrum in deuterochloroform is consistent with the proposed structure; chemical shift in ppm (multiplicity, assignment, relative intensity): 5.35 (apparent triplet, HC=CH, 4 H), 4.13 (triplet, 0-$CH_2$-, 4 H), 3.61 (singlet, O=C-$CH_2$-C=O, 4 H), 2.01, 1.62, and 1.27 (multiplets, -$CH_2$-, 56 H), and 0.88 (triplet, -$CH_3$, 6 H); The product in chloroform-d exists as an equilibrium mixture of keto (about 68%) and enol (about 32%) tautomers which account for additional nmr singlets at 3.22 and 5.18 ppm in ratio of 2:1, respectively.

Dioleyl 3-hydroxyglutarate. Sodium borohydride (46.36 g, 1.22 mole) in 800 mL of ice water and dioleyl 3-oxoglutarate (525.0 g, about 0.81 mole) in 800 mL diethyl ether are combined in a 3000 mL flask containing a magnetic stirrer bar and the two phase mixture is stirred vigorously at ambient temperature for 22.5 hours. The ether layer is separated, washed twice with 800 mL portions of 5% aqueous HCl, and washed twice with 800 mL portions of water. The ether solution is evaporated on a vacuum rotary evaporator to give 504.3 g (about 96% yield) of straw colored oil. Excess oleyl alcohol is removed by passage of the crude product through a falling film still (168 degrees, 0.8 mmHg). An nmr spectrum of the final product is consistent with the title structure; chemical shift in ppm (multiplicity, assignment, relative intensity): 5.35 (apparent triplet, HC=CH, 4

H), 4.46 (quintet of doublets, methine H, 1 H), 4.10 (triplet, O-$CH_2$-, 4 H), 3.45 (doublet, -O-H, 1 H), 2.55 (doublet, HO-C-$CH_2$-C=O, 4 H), 2.00, 1.63, and 1.26 (multiplets, -$CH_2$-, 56 H), and 0.88 (triplet, -$CH_3$, 6 H).

1:2 Adduct of Adipoyl chloride and Dioleyl 3-hydroxyglutarate. Dioleyl 3-hydroxyglutarate (32.45 g, 0.05 mole) and adipoyl chloride (4.58g, 0.025 mole) are combined in a 100 mL flask containing a magnetic stir bar and sealed with a stopcock. The mixture is heated with stirring at 110-115 degrees C for 24 hours under 125 mmHg vacuum. The reaction mixture at this point exhibits a weight loss which is consistent with the formation of HCl as a volatile reaction by-product. The nmr spectrum of the isolated, red orange oil in deuterochloroform is consistent with the 1:2 adduct structure; chemical shift in ppm (multiplicity, assignment, relative intensity): 5.51 (quintet, methine H, 2H), 5.35 (apparent triplet, HC=CH, 8 H), 4.07 (triplet, O=C-O-$CH_2$-, 8 H), 2.70 (doublet, HO-C-$CH_2$-C=O, 8 H), 2.29 (apparent triplet, O=C-$CH_2$- of adipate bridge, 4 H), 2.01, 1.62 and 1.31 (multiplets, -$CH_2$-, 116 H) and 0.89 (triplet, -$CH_3$, 12 H).

## EXAMPLE 2

The process of Example 1 is repeated but this time employing glutaric anhydride and two equivalents of dioleyl 2-hydroxymethylmalonate to form a novel complex linked ester having the structural formula: $CH_2[CH_2CO_2CH(CO_2CH_2(CH_2)_7CH=CH(CH_2)_7CH_3)_2]_2$

## EXAMPLE 3

By essentially the same procedure as detailed in Example 1, sebacoyl chloride is reacted with two equivalents of dioleyl 2-hydroxymethylsuccinate to form a novel complex linked ester with a molecular formula of $C_{51}H_{91}O_7$.

## EXAMPLE 4

Filled Cream. About 18Kg of a fat mimetic (mp 32 to 35 degrees C) of Example 3 is homogenized with 82 Kg of skim milk in a conventional dairy homogenizer to afford a "filled cream" composition.

## EXAMPLE 5

Ice Cream. The "filled cream" composition of Example 4 (68 parts) is combined with 15 parts condensed skim milk, 15 parts sugar, 0.5 parts gelatin, 1.0 part flavor, and 0.25 parts color to produce an ice cream mix which is processed in the normal manner to yield a modified ice cream product.

## EXAMPLE 6

Filled Milk. About 100 parts of the filled cream composition prepared in Example 4 is combined with about 620 parts of skim milk to prepare a "filled milk" composition.

## EXAMPLE 7

Cheese Products. The filled milk product obtained in Example 6 is treated like natural milk in the normal cheese making process (as is practiced, for example in the production of cheddar or swiss cheese). Preferably 10% butter oil is added to the fat mimetic portion of the filled milk product before it is employed in the process to enhance the proper flavor development of the cheese products.

## EXAMPLE 8

Butter cream icing is prepared by blending:

| Ingredient | g. |
|---|---|
| Sugar | 227.0 |
| Fat mimetic of Example 2 | 70.8 |
| Water | 28.4 |
| Non-Fat Dry Milk | 14.0 |
| Emulsifier (used with di-alkyl glycerol ether blend only) | 1.4 |
| Salt | 1.0 |
| Vanilla | 1.0 |

All of the ingredients are creamed in a mixer at medium speed.

## EXAMPLE 9

Vanilla Wafers. Twenty-five parts of a (plastic) fat mimetic are blended with 100 parts flour, 72 parts granulated sugar, 5 parts high fructose corn syrup, 1 part non-fat dry milk, 1 part salt, 1/10 part ammonium bicarbonate, 1 part dried egg yolk, 1/10 part sodium bicarbonate, and 55 parts water. The dough so formed is rolled, wire cut to 1/4 inch thickness, and baked by the usual process to give a vanilla wafer cookie.

## EXAMPLE 10

This example shows the preparation, from intermediate to final molecular composition, of a fat mimetic material of the invention containing a linking dibasic acid unit and having the following structure:

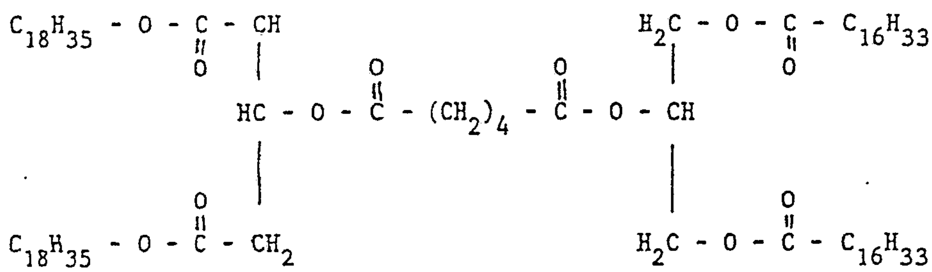

Mono-(dioleyl glutar-3-yl) succinate. A 500-mL round bottom flask, containing a magnetic stirrer bar, is charged with 62.2g (0.096 mole) dioleyl 3-hydroxyglutarate, 13.8 g (0.14 mole) succinic anhydride, 3.2 g (0.026 mole) 4-(dimethylamino)pyridine and 200 mL of anhydrous pyridine. The resulting solution is stirred at ambient temperature for three days, and is poured into 1500 mL of water. This mixture is extracted with 4 x 400 mL portions of diethyl ether, and the combined extracts are washed with 5% aqueous HCl, and are dried over anhydrous $Na_2CO_3$. After filtration, the volatiles are removed on a vacuum rotary evaporator (45 degrees C and 100 Torr, then ambient temperature and 2 Torr). The yield of yellow oil is 58g (82% of theory). The product is characterised by IR and NMR spectroscopy: IR, neat: 3.33-3.45 microns (very broad, -OH); 5.76 (strong, C=O); 6.29 (weak, C=C); 8.62 (strong, C-O); NMR, chloroform-d: 0.88 ppm (triplet, 6H, $-CH_3$); 1.30 (multiplet, 40H, $-CH_2-$); 1.61 (quintet, 4H, $-O-CH_2-CH_2-CH_2-$); 2.01 (multiplet, 8H, $C=C-CH_2-$); 2.65 (apparent doublet of doublets, 4H, $O=C-CH_2-CH_2-C=O$); 2.71 (doublet, 4H, $O=C-CH_2-CH-CH_2-C=O$); 5.35 (multiplet, 4H, HC=CH); and 5.53 (quintet, 1H, $-CH_2-CH-CH_2-$).

Elemental Analysis:

| | | | | |
|---|---|---|---|---|
| Calc. for $C_{45}H_{80}O_8$, FW 749.12: | C | 72.15, | H | 10.76; |
| Found: | C | 72.15, | H | 10.87. |

Mono-(dioleyl glutar-3-yl) succinoyl monochloride. A portion of the above acid ester (58.0g, ~0.0793 mole) and 200 mL of thionyl chloride are charged to a 500-mL round bottom flask, containing a magnetic stirrer bar,

and fitted with a drying tube filled with Drierite. The reaction mixture is stirred at room temperature for 24 hours, whereupon the volatiles are removed on a vacuum rotary evaporator (60 degrees C and 100 Torr) to afford 59g (99%) of a darm brown, viscous oil, whose structure is supported by its NMR spectrum: NMR, chloroform-d: 0.88 ppm (triplet, 6H, $-CH_3$); 1.30 (multiplet, 40H, $-CH_2-$); 1.61 (quintet, $-O-CH_2-CH_2-CH_2-$); 2.01 (multiplet, 8H, $C=C-CH_2-$); 2.65 and 3.20 (apparent triplets, 2H and 2H, $O=C-CH_2-CH_2-C=O$); 2.72 (doublet, 4H, $O=C-CH_2-CH-CH_2-C=O$); 4.07 (triplet, 4H, $-O-CH_2-$); 5.35 (multiplet, 4H, $HC=CH$); and 5.56 (quintet, 1H, $-CH_2-CH-CH_2-$).

Elemental Analysis:

| | | | | | | |
|---|---|---|---|---|---|---|
| Calc. for $C_{45}H_{79}C_{107}$, FW 767.57: | C | 70.42, | H | 10.37, | Cl | 4.62; |
| Found: | C | 67.69, | H | 10,01, | Cl | 7.30. |

The elemental analysis, including the higher than expected value determined for chlorine is consistent with product contamination with 0.57 wt% (or 4.1 mole%) thionyl chloride. <u>One-to-one adduct between mono-(dioleyl glutar-3-yl) succinoyl mono-chloride and 1,3-dipalmitoyl glycerol</u>. In a dry, 250-mL, single neck flask containing a magnetic stirrer bar and fitted with a thermometer and a vacuum stopcock are combined 56.9g (0.1 mole) 1,3-dipalmitoyl glycerol and a 76.8g portion (0.1 mole) of mono-(dioleyl glutar-3-yl) succinoyl monochloride prepared above. The flask is sealed and placed under vacuum while being heated to 110 degrees C by means of a heating mantle. The mixture is heated with stirring for sufficient time to yield substantial conversion of reactants to product. After cooling to ambient temperature, the product is characterized by proton NMR spectroscopy (in chloroform-d). The result is consistent with formation of the expected 1:1 adduct between the triester acid chloride and the dipalmitin.

The above description is for the purpose of teaching the person of ordinary skill in the art how to practice the present invention, and it is not intended to detail all those obvious modifications and variations of it which will become apparent to the skilled worker upon reading the description.

## Claims

1. A fat mimetic compound of the following formula:

$$R-(\overset{O}{\overset{\|}{C}}-O-R')_n$$

wherein the R is a linking covalent bond or aliphatic group; n is 2 to 6; and the R′ groups comprise residues defined by the formula:

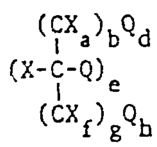

where:
C = a carbon atom;
X = a bridging bonding valence, hydrogen, or substituted or unsubstituted lower aliphatic group (e.g., $C_1$-$C_4$), the various X groups being the same of different;
Q = $-\overset{O}{\overset{\|}{C}}-O-R''$ (carboxylate),
$-R'''-\overset{O}{\overset{\|}{C}}-O-R''$ (alkylcarboxylate),
$-O-\overset{O}{\overset{\|}{C}}-R''$ (carboxy),
$-R'''-O-\overset{O}{\overset{\|}{C}}-R''$ (alkylcarboxy);
$-O-R''$ (alkoxy), or $-R'''-O-R''$ (alkylalkoxy) radicals,
with the proviso that at least one of the Q radicals be other than carboxy;
R″ = Substituted or unsubstituted aliphatic group, containing, for example, no more than 30 carbons, e.g.

$$—(CH_2)_j-\underset{H}{\overset{Z}{\overset{|}{\underset{|}{C}}}}-T$$

the various R′ and R″ groups respectively, being the same or different;
R‴ = Lower alkylene;
T = Hydrogen or a substituted or unsubstituted aliphatic group, e.g., no greater than 22 carbons, containing 0 to 5 unsaturated linkages (e.g., C=C double bonds, C≡C triple bonds) per T residue;
Z = a bridging bond valence, hydrogen, or an alcohol, glycol, ester, ether, or the like, residue;
with the proviso that there is only one bridging bonding valence per R′ group;
and where:
a = 0 to 3;
b = 0 to 4;
d = 1 or 2;
e = 0 to 5;
f = 0 to 3;
g = 0 to 4;
h = 1 or 2;
j = 0 to 10.

2. A compound according to claim 1 wherein each R′ group contains from 1 to 3 Q radicals.

3. A compound according to claim 2 wherein each R′ group contains 2 Q radicals.

4. A compound of claim 1 wherein n is 2.

5. A compound according to claim 4 wherein there are a total of four Q radicals, two on each of the two R′ groups.

6. A compound of claim 1 wherein n is 3.

7. A compound according to claim 6 wherein there are a total of six Q radicals, two on each of the three R′ groups.

8. A compound according to claim 1 wherein at least one Q comprises carboxylate or alkylcarboxylate.

9. A compound according to claim 1 wherein at least one Q comprises carboxy or alkylcarboxy.

10. A compound according to claim 1 wherein at least one Q comprises alkoxy or alkylalkoxy.

11. A compound according to claim 1 wherein R comprises an aliphatic group containing up to 10 carbons.

12. A compound according to claim 11 wherein R comprises an aliphatic group containing from 1 to 6 carbons.

13. A compound according to claim 12 wherein R is a saturated aliphatic group containing 4 carbons.

14. A compound according to claim 13 wherein there are a total of four Q radicals, two on each of R′ groups.

15. A compound according to claim 14 wherein at least two of the Q radicals are carboxy or alkylcarboxy radicals of the formula

$-O-\overset{O}{\overset{\|}{C}}-R''$ or

$R'''-O-\overset{O}{\overset{\|}{C}}-R''$ wherein

$-O-\overset{O}{\overset{\|}{C}}-R''$ is the residue of a fatty acid derived from natural fat.

16. A compound according to claim 15 wherein all four Q radicals are as defined in claim 15, with the proviso that at least one is alkylcarboxy.

17. A compound according to claim 15 wherein at least one Q radical is a carboxylate or alkylcarboxylate radical of the

$-\overset{O}{\overset{\|}{C}}-O-R''$ or

$-R'''-\overset{O}{\overset{\|}{C}}-O-R''$ wherein

$-\overset{O}{\overset{\|}{C}}-O-R''$ is the residue of a fatty alcohol.

18. A compound according to claim 1 which contains at least one R‴ as methylene.

19. A compound according to claim 1 wherein:
a = 0 to 2,
b = 0 to 1,
e = 1 to 2,
f = 0 to 2,

g = 0 to 1, and
j = 0 to 3.

20. A food product containing a fat mimetic compound according to any one of claims 1 to 19, in an amount sufficient to provide a fat mimetic effect attributable to said compound.

21. A food product containing a fat mimetic compound according to claim 15.

22. A food product containing a fat mimetic compound according to claim 17.